# EUROPÄISCHE PATENTSCHRIFT

(11) **EP 2 852 363 B1**
(45) Veröffentlichungstag und Bekanntmachung des Hinweises auf die Patenterteilung: **14.09.2016**
(21) Anmeldenummer: 13732555.1
(22) Anmeldetag: 01.07.2013
(51) Int. Cl.: A61G 13/10, F16M 11/04, A61B 90/00, B25B 1/08

(54) **BEFESTIGUNGSEINRICHTUNG ZUM BEFESTIGEN VON ZUBEHÖRTEILEN AN MEDIZINISCHEN EINRICHTUNGEN**
FASTENING DEVICE FOR FASTENING ACCESSORY PARTS TO MEDICAL DEVICES
DISPOSITIF DE FIXATION SERVANT À FIXER DES ACCESSOIRES SUR DES INSTALLATIONS MÉDICALES

(30) Priorität: 02.07.2012 DE 102012211438
(43) Veröffentlichungstag der Anmeldung: 01.04.2015
(73) Patentinhaber: TRUMPF Medizin Systeme GmbH + Co. KG, 07318 Saalfeld (DE)
(72) Erfinder: MUELLER, Ivo, 07318 Saalfeld (DE); FIEDLER, Kevin, 07768 Kahla (DE)
(74) Vertreter: Prüfer & Partner mbB Patentanwälte · Rechtsanwälte
(86) Internationale Anmeldenummer: PCT/EP2013/063819
(87) Internationale Veröffentlichungsnummer: WO 2014/005989

(56) Entgegenhaltungen:
- DE-A1- 3 628 911
- DE-U1- 9 202 090
- GB-A- 2 410 289
- US-A- 4 018 412
- US-A- 5 169 106
- US-A- 5 320 314
- US-A1- 2004 034 932

## Beschreibung

Die Erfindung betrifft eine Befestigungseinrichtung zum Befestigen von Zubehörteilen an medizinischen Einrichtungen, insbesondere eine Befestigungseinrichtung zum Befestigen von Zubehörteilen an Halteeinrichtungen von medizinischen. Einrichtungen, die sich in ihren Abmessungen unterscheiden.

Es sind Befestigungseinrichtungen für Zubehörteile an medizinischen Einrichtungen bekannt, mit denen beispielsweise ein Anästhesiebogen an einen Operationstisch oder Infusionsstangen an den Operationstisch oder an Deckenversorgungseinrichtungen angebracht werden können. Die medizinischen Einrichtungen besitzen dafür als Halteeinrichtungen meist so genannte Normschienen, Stangen, oder ähnliche Halteeinrichtungen, die aber regional unterschiedlichen Normen unterliegen. Die Normschienen haben zwar einen im Wesentlichen rechteckigen Querschnitt aber unterschiedliche Nennmaße. Darüber hinaus sind die Maße der Normschienen mit großen Toleranzen behaftet.

Daher ist es bisher erforderlich, für Zubehörteile, Befestigungseinrichtungen mit unterschiedlichen Abmessungen für die unterschiedlichen Normschienen anzufertigen, was aber einen erhöhten Aufwand in Logistik und Lagerhaltung bedeutet, wobei ein Anbringen der Befestigungseinrichtungen aufgrund der großen Toleranzen trotzdem mit einen hohen Bedienungsaufwand verbunden ist.

Dokument US 4,018,412 A offenbart dazu einen Halter für einen Operationstisch, der eine erste unbewegliche und zweite in eine Klemmrichtung bewegliche Klaue aufweist. Eine Antriebsvorrichtung für die zweite Klaue weist eine Steuerkulisse mit einer Steuerfläche auf, wobei sich die Steuerkulisse an einer Abstützeinrichtung abstützt. Die Steuerfläche weist einen ersten Bereich auf, der mit der Klemmrichtung einen ersten Winkel einschließt, und einen sich direkt an den ersten Bereich anschließenden zweiten Bereich auf, der mit der Klemmrichtung einen zweiten Winkel einschließt.

Der Erfindung liegt die Aufgabe zu Grunde, diese Nachteile zu eliminieren, und eine Befestigungseinrichtung bereitzustellen, die unabhängig von der regionalen Ausführung und der Toleranzen der Halteeinrichtung ein schnelles und sicheres Befestigen der Befestigungseinrichtung an der Halteeinrichtung gewährleistet.

Die Aufgabe wird durch den Gegenstand gemäß Anspruch 1 gelöst. Weiterentwicklungen der Erfindung sind Gegenstand der Unteransprüche.

Gemäß einem Aspekt der Erfindung kann die Befestigungseinrichtung mit einer Steuerkulisse versehen sein, die eine Steuerfläche mit zwei Bereichen aufweist, welche mit einer Klemmrichtung einer beweglichen Klaue unterschiedliche Winkel einschließen. Dadurch kann sowohl ein schnelles Zustellen um einen größeren Zustellweg einer Befestigungseinrichtung, also ein kurzer Bedienweg zum Spannen, als auch ein sicheres Klemmen mit einer minimalen Bewegung realisiert werden. Damit wird der Vorteil erzielt, dass es nicht erforderlich ist, für verschiedene Halteeinrichtungen verschiedene Befestigungseinrichtungen vorzusehen.

Die Erfindung wird nun anhand von Ausführungsbeispielen unter Bezugnahme auf die beigefügten Zeichnungen erläutert.
- Fig. 1A: zeigt eine Seitenansicht einer prinzipiellen Darstellung einer Befestigungseinrichtung in einer ersten Ausführungsform;
- Fig. 1B: zeigt eine Vorderansicht der prinzipiellen Darstellung der Befestigungseinrichtung in der ersten Ausführungsform;
- Fig. 2: ist eine perspektivische Explosionsdarstellung der Befestigungseinrichtung in einer zweiten Ausführungsform;
- Fig. 3A: ist eine Vorderansicht einer Steuerkulisse der Befestigungseinrichtung in der zweiten Ausführungsform;
- Fig. 3B: ist eine Draufsicht auf die Steuerkulisse aus Fig. 3A; und
- Fig. 4: ist eine perspektivische Explosionsdarstellung der Befestigungseinrichtung in einer dritten Ausführungsform.

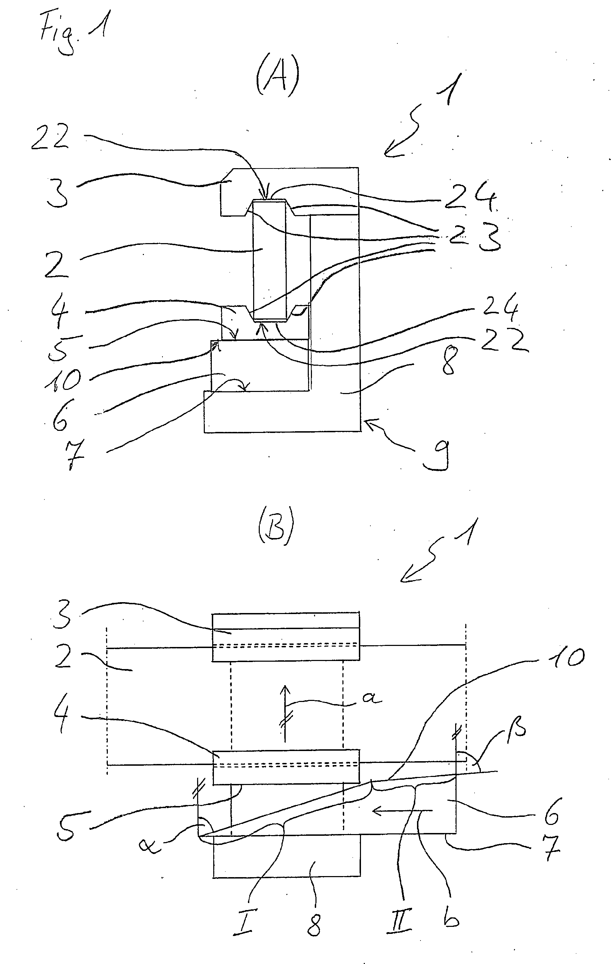

Fig. 1 zeigt eine prinzipielle Darstellung einer Befestigungseinrichtung 1 zur Befestigung von Zubehörteilen an medizinischen Einrichtungen in einer ersten Ausführungsform. In Fig. 1(A) ist eine Seitenansicht der Befestigungseinrichtung 1 dargestellt und in Fig. 1(B) ist eine Vorderansicht der Befestigungseinrichtung 1 dargestellt, wobei die Befestigungseinrichtung 1 eine Normschiene 2 einklemmt, und somit an ihr befestigt ist.

Die Befestigungseinrichtung 1 weist eine erste Klaue 3 auf, die zu der Befestigungseinrichtung 1 unbeweglich ist. Weiterhin weist die Befestigungseinrichtung 1 eine zweite Klaue 4 auf, die zu der ersten Klaue 3 in einer Klemmrichtung a bewegliche ist, um z.B. die Normschiene 2 zu klemmen.

Zum Bewegen der zweiten Klaue 4 ist eine Antriebseinrichtung, bestehend aus einer Abstützeinrichtung 9 und einer Steuerkulisse 6, vorgesehen, wobei sich die Steuerkulisse 6 über eine Abstützfläche 7 in Bezug auf die erste Klaue 3 an einem Haltewinkel 8 abstützt. Die Abstützfläche 7 und der Haltewinkel 8 bilden zusammen die Abstützeinrichtung 9. Eine Distanz zwischen der ersten Klaue 3 und der Abstützfläche 7 ist daher in dieser Ausführungsform konstant.

Die zweite, bewegliche Klaue 4 ist mittels einer nicht gezeigten Führung in der Klemmrichtung a beweglich. An der der Steuerkulisse 6 zugewandten Seite weist die zweite Klaue 4 eine Wirkfläche 5 auf, über die die zweite Klaue 4 bewegbar ist.

Die Steuerkulisse 6 weist an der der Abstützfläche 7 abgewandten Seite eine Steuerfläche 10 auf. Die Steuerfläche 10 wirkt mit der Wirkfläche 5 zusammen, um bei einer Bewegung der Steuerkulisse 6 in einer Betätigungsrichtung b eine Bewegung der zweiten Klaue 4 in der Klemmrichtung a zu veranlassen.

Die Steuerfläche 10 ist in zwei Bereiche unterteilt. Ein erster Bereich I schließt mit der Klemmrichtung a einen ersten Winkel α ein. Ein zweiter Bereich II schließt mit der Klemmrichtung a einen zweiten Winkel β ein. Die Winkel α, β sind so gewählt, dass der Schenkel in Richtung der Klemmrichtung a jeweils als mit der Klemmrichtung a parallel dargestellt ist. Der Winkel α ist kleiner als der Winkel β, wodurch bei einer gleichmäßigen Bewegung der Steuerkulisse 6 in der Betätigungsrichtung b die Bewegung der zweiten Klaue 4 in der Klemmrichtung a in dem ersten Bereich I schneller als in dem zweiten Bereich II ist. Optional sind die Winkel α, β so ausgelegt, dass der Winkel α nicht selbsthemmend ist, d.h., dass durch eine Kraft auf die zweite Klaue 4 entgegen der Klemmrichtung a eine Bewegung der Steuerkulisse 6 verursacht wird, wohingegen der Winkel β selbsthemmend ist, d.h., dass durch eine Kraft entgegen der Klemmrichtung a auf die zweite Klaue 4 keine Bewegung der Steuerkulisse 6 verursacht werden kann. Die Bestimmung der Winkel für Selbsthemmung ist in erster Linie von den Material- und O-berflächeneigenschaften abhängig.

In alternativen Ausführungsformen weist die Steuerfläche 10 zusätzlich zu einem ersten Abschnitt mit dem ersten Bereich I und dem zweiten Bereich II mindestens einen weiteren Abschnitt mit jeweils zumindest einem funktionell identischen ersten Bereich I' und einem funktionell identischen zweiten Bereich II auf. Die Abschnitte sind so ausgebildet, dass die zweiten Klaue 4 in dem ersten Bereich I, I' und dem sich direkt daran anschließenden zweiten Bereich II, II' im Wesentlichen kontinuierlich in die Klemmrichtung a bewegt wird, wobei alternativ aber auch ein Absatz vorgesehen sein kann, so dass sich die zweite Klaue 4 nach Überwindung eines Abschnitts zunächst wieder entgegen der Bewegungsrichtung a bewegt. Die in den jeweiligen Abschnitten gebildeten Winkel α und β können in jedem Abschnitt gleich sein, aber auch variieren.

Die erste Klaue 3 und die zweite Klaue 4 weisen jeweils eine Nut 22 zum Aufnehmen der Normschiene 2 oder einer alternativen Halteeinrichtung der medizinischen Einrichtung auf. Um Normschienen 2 mit verschiedenen Breiten sicher klemmen zu können, weisen die Nuten 22 im in Fig. 1(A) gezeigten Querschnitt seitliche Flächen 23 auf, die zueinander einen Winkel einschließen, so dass sich ein Abstand zwischen den seitlichen Flächen 23 zu einem Nutgrund 24 hin verringert. Der Nutgrund 24 weist dabei eine Breite auf, die maximal der kleinsten Breite der aufzunehmenden Normschienen 2 entspricht. Die Breite an einem dem Nutgrund 24 gegenüberliegenden Ende muss mindestens der größten Breite der aufzunehmenden Normschienen 2 entsprechen.

Im Betrieb dieser prinzipiellen Befestigungseinrichtung 1 wird die Steuerkulisse 6 in Fig. 1(B) von rechts nach links verschoben. Durch den kleineren ersten Winkel α wird die zweite Klaue 4 bei einem relativ geringen Weg in der Betätigungsrichtung b schnell zu der ersten Klaue 3 bewegt. Dadurch ist ein schnelles Zustellen möglich. Durch den größeren zweiten Winkel β kann bei einer relativ kleinen Kraft in der Betätigungsrichtung b die zweite Klaue 4 mit einer großen Kraft zu der ersten Klaue 3 hin bewegt werden, so dass die Normschiene 2 sicher geklemmt wird.

Bei einer nicht selbsthemmenden Auslegung des ersten Winkels α wird ein selbsttätiges Zurückstellen der zweiten Klaue 4 in eine Position, die möglichst weit von der ersten Klaue 3 entfernt ist, ermöglicht, wodurch ein leichtes Einführen der Normschiene 2 bei einem nächsten Klemmvorgang möglich wird.

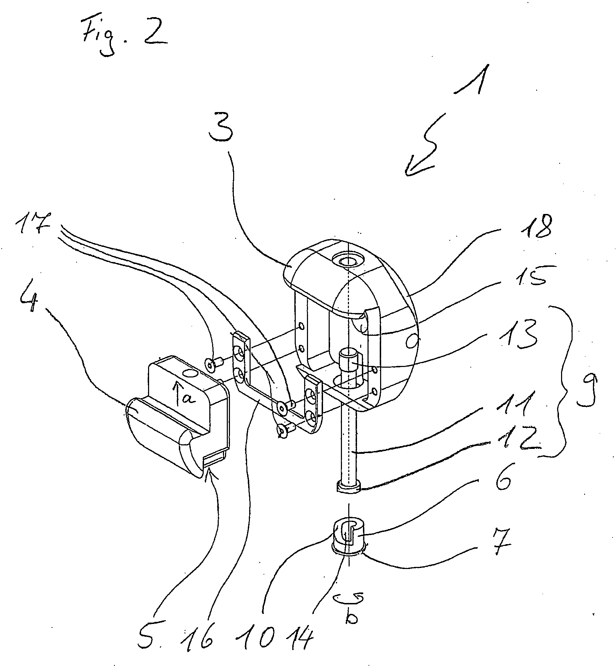

Bei einer selbsthemmenden Auslegung des zweiten Winkels β ist es nicht erforderlich, die Steuerkulisse 6 gegen eine Bewegung entgegen der Betätigungsrichtüng b zu sichern, wobei trotzdem eine sichere Klemmung der Normschiene 2 gewährleistet ist. Fig. 2 zeigt eine perspektivische Explosionsdarstellung der Befestigungseinrichtung 1 in einer zweiten Ausführungsform. Der wesentliche Unterschied zu der ersten, prinzipiellen Ausführungsform ist die Gestalt der Steuerkulisse 6. In dieser Ausführungsform ist die Steuerkulisse 6 im Wesentlichen zylindrisch ausgebildet: Die zylindrische Steuerkulisse 6 wird nachstehend im Detail beschrieben. Die Steuerkulisse 6 weist die Steuerfläche 10 umlaufend an einer axialen Stirnseite davon auf. Die Steuerkulisse 6 ist gegenüber der Wirkfläche 5, die sich an der Unterseite der zweiten Klaue 4 befindet, verdrehbar ausgebildet.

In dieser Ausführungsform weist die Befestigungseinrichtung 1 einen Bolzen 11 auf. Der Bolzen 11 steht mit der ersten Klaue 3 in Verbindung. Der Bolzen 11 weist ein Gewinde als ein Verbindungselement 13 auf, das direkt mit einem in der ersten Klaue 3 vorgesehenen Gewinde verbunden ist und den Bolzen 11 an der ersten Klaue 3 verschraubbar fixiert. Alternativ ist eine Verbindung zu der ersten Klaue 3 über weitere Bauteile vorgesehen und/oder der Bolzen 11 ist mit der ersten Klaue 3 axial drehbar verbunden.

Der Bolzen 11 weist weiterhin einen Absatz 12 auf. Alternativ kann auch eine separate Scheibe oder eine separate Buchse vorgesehen sein, die mit dem Bolzen 11 so verbunden ist, dass sich ein entsprechender Absatz bildet. Eine der ersten Klaue 3 zugewandte Fläche des Absatzes 12 bildet hier eine Fläche, an der sich die Abstützfläche 7 der Steuerkulisse 6 abstützen kann. Durch eine feste Verbindung zwischen dem Bolzen 11 und der ersten Klaue 3 über das Verbindungselement 13 und durch das Vorsehen des Absatzes 12 wird ein vorbestimmter Abstand in der Klemmrichtung a zwischen der ersten Klaue 3 und der Abstützfläche 7 nicht überschritten. Der vorbestimmte Abstand ist optional über Distanzscheiben oder Einstellschrauben anforderungsgerecht veränderbar. Somit wird über die Steuerkulisse 6 und den Bolzen 11 mit der Fläche des Absatzes 12 die Antriebsvorrichtung gebildet. Die Steuerkulisse 6 ist hier gegenüber dem Bolzen 11 drehbar.

Als Alternative sind nicht zwingend ein Bolzen und ein Bohrung in der Steuerkulisse vorgesehen, sondern die Steuerkulisse kann auf eine andere Weise, z.B. in einer mit der ersten Klaue 3 verbundenen Senkung drehbar gelagert sein.

Die Befestigungseinrichtung 1 weist eine Befestigungsbohrung 15 auf, über die das Zubehörteil an der Befestigungseinrichtung 1 angebracht wird.

Ein U-förmiger Aushängeschutz 16 ist mit mehreren (hier vier) Schrauben an einem Gehäuse 18 der Befestigungseinrichtung 1 angebracht. Durch den Aushängeschutz 16 wird die Befestigungseinrichtung 1 daran gehindert, durch eine Kraft lediglich von unten ausgehängt zu werden. Zum Trennen von der Normschiene 2 muss die Befestigungseinrichtung 1 erst gekippt und dann ausgehängt werden. Dadurch wird ein versehentliches Aushängen, beispielsweise durch einen Schlag von unten, verhindert.

Im Betrieb wird, im Unterschied zu der ersten Ausführungsform, die Steuerkulisse 6 nicht verschoben, sondern gedreht. Durch die Drehung übertragen, wie später beschrieben, die zwei Bereiche I und II der Steuerfläche 10 der Steuerkulisse 6 die Bewegung auf die zweite Klaue 4. Die Drehung wird über eine nicht gezeigt Betätigungsvorrichtung in die Steuerkulisse 6 eingeleitet.

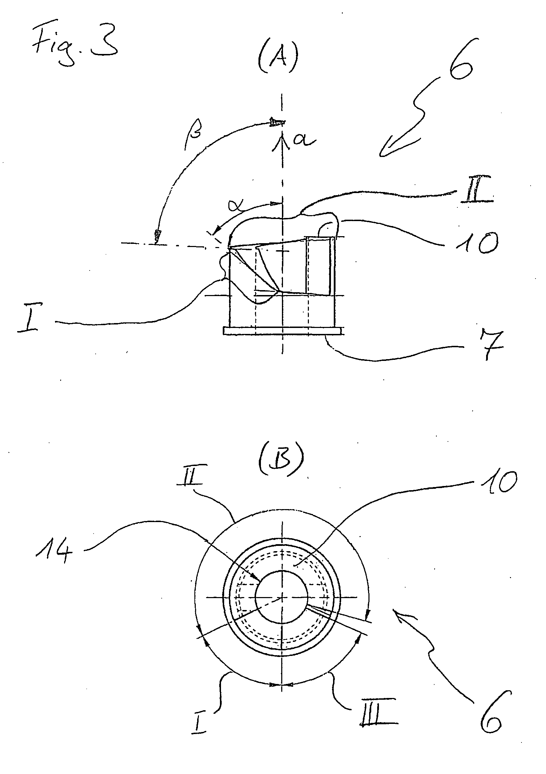

Fig. 3(A) und 3(B) sind Darstellungen der Steuerkulisse 6 der Befestigungseinrichtung 1 in der zweiten Ausführungsform. Fig. 3(A) ist eine Vorderansicht der Steuerkulisse 6 und Fig. 3(B) ist eine Draufsicht darauf. Unter Verwendung dieser Figuren wird die Steuerkulisse 6 im Detail beschrieben.

Die Steuerkulisse 6 ist zylindrisch ausgeführt und weist die Steuerfläche 10 an einer der axialen Stirnseiten (hier der oberen Stirnseite) auf. An der gegenüberliegenden Stirnseite (hier der unteren Stirnseite) ist die Abstützfläche 7 vorgesehen.

Die Stirnfläche, an der die Steuerfläche 10 vorgesehen ist, ist umlaufend so ausgebildet, dass der erste Bereich I und der zweite Bereich II vorgesehen sind. Die Steuerfläche 10 weist im ersten Bereich I auch hier gegenüber der Klemmrichtung a einen Winkel α auf. Der Winkel beträgt in dieser Ausführungsform 45°. In alternativen Ausführungsformen liegt er in einem Winkelbereich von 35° bis 55°. Durch die Auswahl dieses Bereichs für den Winkel α ist gewährleistet, dass der Winkel nicht selbsthemmend ist.

Der erste Bereich I weist einen Winkel von im Wesentlichen 64° über den Umfang der Steuerkulisse 6 auf. Dieser Umfangswinkel kann alternativ auch in einem anderen Größenbereich gewählt werden, wobei ein Zustellweg und eine Betätigungskraft für die Zustellung wesentliche Parameter sind.

Die Steuerfläche weist im zweiten Bereich II hier einen Winkel β von im Wesentlichen 85,7° auf. In alternativen Ausführungsformen liegt er in einem Winkelbereich von 80° bis 89°. Durch die Auswahl dieses Bereichs für den Winkel β ist gewährleistet, dass der Winkel selbsthemmend ist.

Der zweite Bereich II weist einen Winkel von im Wesentlichen 220° über den Umfang der Steuerkulisse 6 auf. Dieser Umfangswinkel kann alternativ auch in einem anderen Größenbereich gewählt werden, wobei ein Klemmweg und eine Betätigungskraft für das Klemmen wesentliche Parameter sind. Günstigerweise ist der Umfangswinkel des ersten Bereichs I kleiner als der Umfangswinkel des zweiten Bereichs II, da auf diese Weise das Aufbringen einer sicheren Klemmkraft gewährleistet ist.

Zwischen dem ersten Bereich I und dem zweiten Bereich II weist die Steuerfläche 10 noch einen dritten Bereich III auf. Dieser dritte Bereich III ist so gestaltet, dass durch die Steuerfläche 10 auf die Wirkfläche 5 der zweiten Klaue 4 (Fig. 2) keine Kraft ausgeübt wird, und somit keine Bewegung in der Klemmrichtung a vorliegt. Dieser Bereich ist lediglich für das Aufnehmen der Wirkfläche 5, die hier annähernd zapfenförmig gestaltet ist und von der zweiten Klaue nach unten vorsteht, in einem Zustand, in dem die zweite Klaue 4 nicht bewegt ist, vorgesehen.

Die Steuerkulisse 6 weist eine Durchgangsbohrung 14 auf, deren Durchmesser auf den Durchmesser des in Fig. 2 gezeigten Bolzens 11 abgestimmt ist.

Ebenso wie in der ersten Ausführungsform kann hier die Steuerfläche 10 alternativ auch mehrere mindestens einen ersten Bereich I, I' und einen zweiten Bereich II, II aufweisende Abschnitte umfassen, wobei sich die Umfangswinkel dann entsprechend verringern.

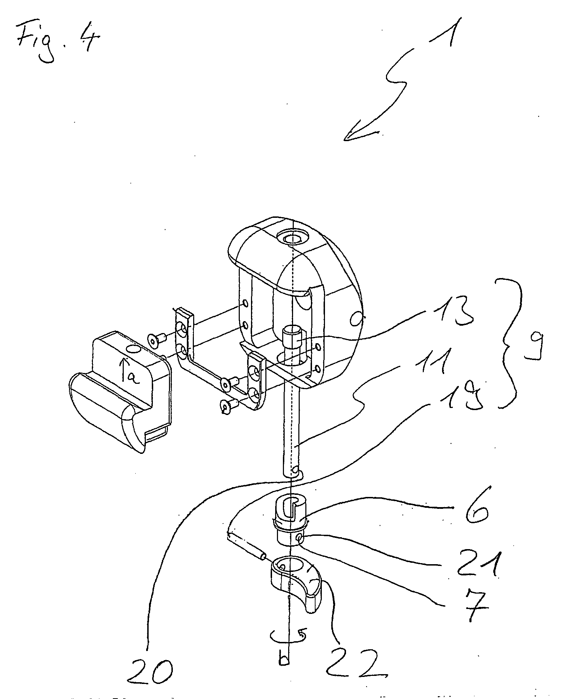

Fig. 4 ist eine perspektivische Explosionsdarstellung der Befestigungseinrichtung 1 in einer dritten Ausführungsform. Der Unterschied zu der Befestigungseinrichtung 1 in der zweiten Ausführungsform ist im Wesentlichen die Ausbildung der Antriebseinrichtung bestehend aus der Steuerkulisse 6 und der Abstützeinrichtung 9.

Die Abstützeinrichtung 9 besteht hier im Wesentlichen aus dem Bolzen 11 mit dem Verbindungselement 13 und dem Stift 19.

Das Verbindungselement 13 verbindet auch hier den Bolzen 11 mit der ersten Klaue 3, jedoch wird der Bolzen 11 dadurch nicht fixiert, sondern das Verbindungselement 13 wird durch ein Bewegungsgewinde mit einer vorbestimmten Gewindesteigung ausgebildet, genauer gesagt ist ein Ende des Bolzens 11 mit einem Außengewinde versehen und die erste Klaue 3 weist ein entsprechendes Innengewinde auf.

Durch das Vorsehen des Bewegungsgewindes zwischen dem Bolzen 11 und der ersten Klaue 3 über das Verbindungselement 13 und durch das Vorsehen des Stifts 19 in der Querbohrung 20 des Bolzens 11 ist ein vorbestimmter Abstand zwischen der ersten Klaue 3 und der Abstützfläche 7 in der Klemmrichtung a durch Drehen des Bolzens veränderbar.

Die Steuerungskulisse 6 ist hier mittels des Stifts 19 mit dem Bolzen 11 über eine Bohrung 20 des Bolzens fest verbunden. Die Abstützfläche 7 der Steuerungskulisse 6 ist hier die innere Umfangsfläche einer Querbohrung 21 der Steuerungskulisse 6. Die Umfangsfläche der Querbohrung 21 stützt sich an dem Stift 19 der Abstützeinrichtung 9 der Antriebseinrichtung ab.

Vorteilhafterweise ist ein Betätigungshebel ebenfalls über den Stift 19 mit der Steuerkulisse 6 und dem Bolzen 11 verbunden.

Die vorbestimmte Gewindesteigung ist so ausgelegt, dass sie einen selbsthemmenden Steigungswinkel aufweist. Damit ist es auch hier nicht notwendig, den Bolzen 11 gegen ein Verdrehen zu sichern.

Durch die Gewindesteigung und den ersten Winkel α wird im ersten Bereich I ein sehr schneller Hub, der sich aus der Bewegung durch das Bewegungsgewinde und durch die Steuerkulisse zusammensetzt, erreicht. Im zweiten Bereich II wird durch den selbsthemmenden zweiten Winkel β und die selbsthemmende Gewindesteigung ein kraftvolles, selbsthemmendes Verspannen gegen die Normschiene möglich.

Ferner sind der Steigungswinkel des Verbindungselements 13 und der zweite Bereich II der Steuerfläche 10 der Steuerkulisse 6 so aufeinander abgestimmt, dass ein durch ein Drehen des Bolzens 11 um einen verbleibenden Winkelbereich außerhalb des zweiten Bereichs II infolge der Gewindesteigung des Befestigungselements 13 verursachter Weganteil in der Klemmrichtung a maximal einem Weg der Steuerfläche 10 in der Klemmrichtung a entlang dem zweiten Bereich II entspricht. Solange dieser Weganteil kleiner als dieser Weg ist, ist gewährleistet, dass ein Startpunkt einer selbsthemmenden Klemmbewegung immer tiefer als ein Endpunkt einer vorangegangenen Klemmbewegung ist. Dadurch ist gewährleistet, dass jedes Normschienenmaß selbsthemmend geklemmt werden kann.

Im Betrieb werden der Bolzen 11 und die Steuerkulisse 6 mittels des Betätigungshebels 22 gedreht. Dadurch wird zum einen durch das Bewegungsgewinde der Abstand zwischen der Abstützfläche 7 und der ersten Klaue 3 verringert, also die Steuerkulisse 6 durch das Bewegen des Bolzens 11 in die Klemmrichtung a zu der ersten Klaue 3 hin bewegt, zum anderen wird durch das Verdrehen der Steuerkulisse 6 die zweite Klaue 4 über die Steuerfläche 10 mittels der Wirkfläche 5 zu der ersten Klaue 3 bewegt. Sofern der Weg der zweiten Klaue 3 nicht ausreichend ist, um die Normschiene zwischen der ersten Klaue 3 und der zweiten Klaue 4 zu klemmen, wird der Bolzen 11 weiter gedreht. Bedingt durch das Bewegungsgewinde des Verbindungselements 13 wird somit der vorbestimmte Abstand zwischen der ersten Klaue 3 und der Abstützfläche 7 (der Querbohrung 21) verringert. Somit ist auch der Abstand zwischen der Steuerfläche 10 und der ersten Klaue 3 verringert, so dass, bei einer entsprechenden Abmessung der Normschiene, diese nun geklemmt werden kann. Sollte der Abstand zwischen der ersten Klaue 3 und der zweiten Klaue 4 für ein Klemmen der Normschiene immer noch nicht ausreichend sein, wird der Bolzen 11 mit der Steuerkulisse 6 weiter gedreht, bis der Abstand zwischen der ersten Klaue 3 und der zweiten Klaue 4 ausreichend klein ist, so dass die Normschiene geklemmt wird.

Durch das Auslegen in der Form, dass ein Vorschub bei einem Drehen des Bolzens 11 durch die Gewindesteigung des Befestigungselements 13 um einen verbleibenden Winkelbereich außerhalb des zweiten Bereichs (II) maximal einem Weg der Steuerfläche 10 in der Klemmrichtung a entlang dem zweiten Bereich II entspricht, ist gewährleistet, dass der Abstand, bei dem die Normschiene tatsächlich geklemmt wird, immer in dem Bereich liegt, in dem der zweite Bereich der Steuerfläche 10 mit der Wirkfläche 5 der zweiten Klaue 4 im Eingriff steht.

In einer alternativen Ausführungsform ist der mit dem Bewegungsgewinde versehene Bolzen 11 nicht drehbar. Die Durchgangsbohrung 14 der Steuerkulisse 6 weist hier ein Innengewinde auf, und der Bolzen 11 entlang seiner Längsrichtung zumindest abschnittsweise ein Außengewinde als das Bewegungsgewinde. Bei einer axialen Drehung der Steuerkulisse 6 wird die Steuerkulisse 6 bedingt durch einen Eingriff des Innengewindes der Steuerkulisse 6 in das Bewegungsgewinde des Bolzens 11 an diesem entlang bewegt. Das Bewegungsgewinde ist hier ebenfalls selbsthemmend.

Die verschiedenen angegebenen Ausführungsformen sind, soweit technisch möglich und wirtschaftlich sinnvoll, miteinander kombinierbar.

## Patentansprüche

1. Befestigungseinrichtung (1) zur Befestigung von Zubehörteilen an medizinischen Einrichtungen mit
einer ersten, unbeweglichen Klaue (3),
einer zweiten, zu der ersten Klaue (3) hin bewegbaren Klaue (4), und
einer Antriebseinrichtung (6, 9) zum Bewegen der zweiten Klaue (4) in einer Klemmrichtung (a),
wobei die Antriebseinrichtung (6, 9) eine Steuerkulisse (6) und eine Abstützeinrichtung (9) aufweist,
die Steuerkulisse (6) mit einer Steuerfläche (10) zum Übertragen einer Bewegung der Steuerkulisse (6) in einer Betätigungsrichtung (b) auf eine Bewegung der zweiten Klaue (4) in der Klemmrichtung (a) versehen ist, und
mit einer Abstützfläche (7) versehen ist, und
die Abstützeinrichtung (9) so ausgebildet ist, dass sich die Steuerkulisse (6) in Bezug auf die erste Klaue (3) mit der Abstützfläche (7) an der Abstützeinrichtung (9) abstützt,
wobei die Steuerfläche (10) mit der Klemmrichtung (a) in mindestens einem ersten Bereich (I, I') der Steuerfläche (10) einen ersten Winkel (α) einschließt und in mindestens einem sich direkt an den ersten Bereich (I, I') anschließenden zweiten Bereich (II, II') der Steuerfläche (10) einen zweiten Winkel (β) einschließt, wobei der zweite Winkel (β) größer als der erste Winkel (α) ist, und sich ein Abstand in der Klemmrichtung (a) zwischen der Steuerfläche (10) und der Abstützfläche (7) entlang der Steuerfläche (10) in der Betätigungsrichtung (b) in dem ersten Bereich (I, I') und dem sich direkt daran anschließenden zweiten Bereich (II, II') im Wesentlichen kontinuierlich vergrößert,
**dadurch gekennzeichnet, dass**
der erste Winkel (α) ein nicht-selbsthemmender Winkel ist und der zweite Winkel (β) ein selbsthemmender Winkel ist.

2. Befestigungseinrichtung (1) gemäß Anspruch 1, wobei die Steuerkulisse im Wesentlichen zylindrisch ist, die Steuerfläche umlaufend an einer axialen Stirnseite der Steuerkulisse vorgesehen ist, und die Wirkfläche (5) und die Steuerkulisse zueinander verdrehbar sind.

3. Befestigungseinrichtung (1) gemäß Anspruch 2, wobei die Steuerkulisse (6) eine Bohrung (14) aufweist, und die Antriebseinrichtung einen mit der ersten Klaue (3) verbundenen Bolzen (11) aufweist, der in der Bohrung (14) aufgenommen ist und um den die Steuerkulisse (6) drehbar ist, und die Abstützeinrichtung (9) mit der ersten Klaue (3) eine Verbindung aufweist, die so ausbildet ist, dass ein vorbestimmter Abstand in der Klemmrichtung (a) zwischen der ersten Klaue (3) und der Abstützfläche (7) nicht überschritten wird.

4. Befestigungseinrichtung (1) gemäß Anspruch 3, wobei die Abstützeinrichtung (9) eine feste Verbindung ist, so dass der Bolzen (11) an der Steuerkulisse (6) fixiert ist und bezüglich der ersten Klaue (3) drehbar ist.

5. Befestigungseinrichtung (1) gemäß Anspruch 3 oder 4, wobei der Bolzen (11) ein Gewinde aufweist, so dass der vorbestimmte Abstand in der Klemmrichtung (a) zwischen der ersten Klaue (3) und der Abstützfläche (7) durch Drehen des Bolzens (11) veränderbar ist.

6. Befestigungseinrichtung (1) gemäß einem der Ansprüche 2 bis 5, wobei der erste Bereich (I) der Steuerfläche (10) einen Umfangswinkel aufweist, der kleiner als ein Umfangswinkel des zweiten Bereichs (II) ist.

7. Befestigungseinrichtung (1) gemäß einem der vorangehenden Ansprüche, wobei der erste Winkel (α) in einem Bereich von 35° bis 55° liegt.

8. Befestigungseinrichtung (1) gemäß Anspruch 7, wobei der erste Winkel (α) im Wesentlichen 45° beträgt.

9. Befestigungseinrichtung (1) gemäß einem der vorangehenden Ansprüche, wobei der zweite Winkel (β) in einem Bereich von 80° bis 89° liegt.

10. Befestigungseinrichtung (1) gemäß Anspruch 9, wobei der zweite Winkel (β) im Wesentlichen 85,7° beträgt

11. Befestigungseinrichtung (1) gemäß Anspruch 5 oder Anspruch 5 und einem der Ansprüche 6 bis 10, wobei das Gewinde eine vorbestimmte Gewindesteigung mit einem selbsthemmenden Steigungswinkel aufweist.

12. Befestigungseinrichtung (1) gemäß Anspruch 11, wobei der Steigungswinkel des Gewindes und der zweite Bereich (II) der Steuerfläche (10) der Steuerkulisse (6) so aufeinander abgestimmt sind, dass ein durch ein Drehen des Bolzens (11) um einen verbleibenden Winkelbereich außerhalb des zweiten Bereichs (II) infolge der Gewindesteigung des Befestigungselements (13) verursachter Weganteil in der Klemmrichtung (a) maximal einem Weg der Steuerfläche (10) in der Klemmrichtung (a) entlang dem zweiten Bereich (II) entspricht

13. Befestigungseinrichtung (1) gemäß einem der vorangehenden Ansprüche, wobei die erste und/oder die zweite Klaue (3, 4) eine Nut (22) zum Aufnehmen einer Halteeinrichtung der medizinischen Einrichtung aufweist, und die Nut (22) im Querschnitt seitliche Flächen (23) aufweist, die zueinander einen Winkel einschließen, so dass sich ein Abstand zwischen den seitlichen Flächen (23) zu einem Nutgrund (24) hin verringert.

## Claims

1. Fastening device (1) for fastening accessory parts to medical devices, the fastening device (1) having
a first fixed claw (3),
a second claw (4) being movable towards the first claw (3), and
a driving device (6, 9) for moving the second claw (4) in a clamping direction (a),
wherein the driving device (6, 9) comprises a motion link (6) and a support device (9),
the motion link (6) is provided with a control surface (10) for transmitting a motion of the motion link (6) in an operating direction (b) into a motion of the second claw (4) in the clamping direction (a), and
it is provided with a support surface (7), and
the support device (9) is configured such that, with respect to the first claw (3), the motion link (6) supports itself against the support device (9) by the support surface (7),
wherein the control surface (10) and the clamping direction (a) confine a first angle (α) in at least a first portion (I, I') of the control surface (10) and they confine a second angle (β) in at least a second portion (II, II') of the control surface being directly adjacent to the first portion (I, I'), wherein the second angle (β) is larger than the first angle (α), and a distance in the clamping direction (a) between the control surface (10) and the support surface (7) along the control surface (10) increases substantially continuously in the first portion (I, I') and the second portion (II, II') directly adjacent thereto in the operating direction (b),
**characterized in that**
the first angle (α) is a non-self-locking angle and the second angle (β) is a self-locking angle.

2. Fastening device (1) according to claim 1, wherein the motion link is substantially cylindrical, the control surface is provided circumferentially at an axial end face of the motion link, and the operating face (5) and the motion link are twistable with respect to another.

3. Fastening device (1) according to claim 2, wherein the motion link (6) comprises a bore (14) and the driving device comprises a bolt (11) connected to the first claw (3), the bolt (11) being accommodated in the bore (14) and the motion link (6) is rotatable about the bolt (11), and the support device (9) comprises a connection to the first claw (3), the connection being configured such that a pre-determined distance in the clamping direction (a) between the first claw (3) and the support surface (7) is not exceeded.

4. Fastening device (1) according to claim 3, wherein the support device (9) is a fixed connection so that the bolt (11) is fixed to the motion link (6) and that the bolt (11) is rotatable with respect to the first claw (3).

5. Fastening device (1) according to claim 3 or 4, wherein the bolt (11) comprises a thread so that the predetermined distance in the clamping direction (a) between the first claw (3) and the support surface (7) is changeable by rotating the bolt (11).

6. Fastening device (1) according to any of the claims 2 to 5, wherein the first portion (I) of the control surface (10) confines an angle of circumference which is smaller than an angle of circumference of the second portion (II).

7. Fastening device (1) according to any of the preceding claims, wherein the first angle (α) is in a range of 35 degrees to 55 degrees.

8. Fastening device (1) according to claim 7, wherein the first angle (α) substantially amounts 45 degrees.

9. Fastening device (1) according to any of the preceding claims, wherein the second angle (β) is in a range of 80 degrees to 89 degrees.

10. Fastening device (1) according to claim 9, wherein the second angle (β) substantially amounts 85.7 degrees.

11. Fastening device (1) according to claim 5, or claim 5 and any of the claims 6 to 10, wherein the thread comprises a predetermined thread pitch having a self-locking pitch angle.

12. Fastening device (1) according to claim 11, wherein the pitch angle of the thread and the second portion (II) of the control surface (10) of the motion link (6) are adjusted to one another such that, due to the thread pitch of the fastening member (13), a portion of a way in the clamping direction (a) being caused by rotating the bolt (11) about a residual portion of an angle outside the second area (II) corresponds to a way of the control surface (10) in the clamping direction (a) along the second area (II).

13. Fastening device (1) according to any of the preceding claims, wherein the first and/or second claw (3, 4) comprises a groove (22) for accommodating a retaining device of the medical device, and, in the cross section, the groove (22) comprises lateral faces (23) comprising an angle with respect to another such that a distance between the lateral faces (23) decreases towards a base of the groove (24).

## Revendications

1. Installation de fixation (1) pour fixer des accessoires sur des installations médicales comportant
- une première pince (3), fixe,
- une seconde pince (4), mobile par rapport à la première pince (3), et
- une installation d'entraînement (6, 9) pour déplacer la seconde pince (4) dans la direction de serrage (a),
- l'installation d'entraînement (6, 9) comportant une coulisse de commande (6) et une installation d'appui (9),
- la coulisse de commande (6) ayant une surface de commande (10) pour transmettre le mouvement de la coulisse de commande (6) dans une direction d'actionnement (b) en un mouvement de la seconde pince (4) dans la direction de serrage (a), et
- une surface d'appui (7), et
- l'installation d'appui (9) est réalisée pour que la coulisse de commande (6) s'appuie par rapport à la première pince (3), avec la surface d'appui (7) contre l'installation d'appui (9),
- la surface de commande (10) faisant avec la direction de serrage (a) un premier angle (α) dans au moins une première zone (I, I') de la surface de commande (10) et dans au moins une seconde zone (II, II') directement adjacente à la première zone (I, I'), de la surface de commande (10), un second angle (β), ce second angle (β) étant plus grand que le premier angle (α) et la distance dans la direction de serrage (a) entre la surface de commande (10) et la surface d'appui (7) le long de la surface de commande (10) dans la direction d'actionnement (b) augmentant pratiquement de manière continue dans la première zone (I, I') et dans la seconde zone (II, II') directement adjacente,
installation **caractérisé en ce que**
le premier angle (α) est un angle non autobloquant et le second angle (β) est un angle autobloquant.

2. Installation de fixation (1) selon la revendication 1,
**caractérisée en ce que**
la coulisse de commande (6) est pratiquement cylindrique, la surface de commande étant prévue sur la face frontale axiale de la coulisse de commande et la surface d'action (5) et la coulisse de commande pouvant tourner l'une par rapport à l'autre.

3. Installation de fixation (1) selon la revendication 2,
**caractérisée en ce que**
la coulisse de commande (6) comporte un perçage (14) et l'installation d'entraînement a un goujon (11) relié à la première pince (3) et logé dans le perçage (14) et pouvant tourner autour de la coulisse de commande (6), et
l'installation d'appui (9) réalise une liaison avec la première pince (3) de façon à ne pas dépasser une distance prédéterminée dans la direction de serrage (a) entre la première pince (3) et la surface d'appui (7).

4. Installation de fixation (1) selon la revendication 3,
**caractérisée en ce que**
l'installation d'appui (9) est une liaison fixe de façon que le goujon (11) soit fixé à la coulisse de commande (6) et puisse tourner par rapport à la première pince (3).

5. Installation de fixation (1) selon la revendication 3 ou 4,
**caractérisée en ce que**
le goujon (11) a un filetage pour modifier la distance prédéfinie dans la direction de serrage (a) entre la première pince (3) et la surface d'appui (7) en tournant le goujon (11).

6. Installation de fixation (1) selon l'une des revendications 2 à 5,
**caractérisée en ce que**
la première zone (I) de la surface de commande (10) s'étend sur un angle périphérique inférieur à l'angle périphérique de la seconde zone (II).

7. Installation de fixation (1) selon l'une des revendications précédentes,
**caractérisée en ce que**
le premier angle (α) est dans une plage comprise entre 35° et 55°.

8. Installation de fixation (1) selon la revendication 7,
**caractérisée en ce que**
le premier angle (α) est pratiquement égal à 45°.

9. Installation de fixation (1) selon l'une des revendications précédentes,
**caractérisée en ce que**
le second angle (β) est dans une plage comprise entre 80° et 89°.

10. Installation de fixation (1) selon la revendication 9,
**caractérisée en ce que**
le second angle (β) est pratiquement égal à 85,7°.

11. Installation de fixation (1) selon la revendication 5 ou selon la revendication 5 et l'une des revendications 6 à 10,
**caractérisée en ce que**
le filetage a un pas défini avec un angle autobloquant.

12. Installation de fixation (1) selon la revendication 11,
**caractérisée en ce que**
l'angle du pas du filetage et la seconde zone (II) de la surface de commande (10) de la coulisse de commande (6) sont déterminés l'un en fonction de l'autre pour que la rotation du goujon (11) dans la plage angulaire résiduelle, au-delà de la seconde plage (II), de sorte que par la pente de l'élément de fixation (13), la partie de trajet générée dans la direction de serrage (a) correspond au maximum à un trajet de la surface de commande (10) dans la direction de serrage (a) le long de la seconde plage (II).

13. Installation de fixation (1) selon l'une des revendications précédentes,
**caractérisée en ce que**
la première et/ ou la seconde pince (3, 4) ont une rainure (22) pour recevoir une installation de maintien de l'installation médicale et la rainure (22) a en section des surfaces latérales (23) faisant entre elles un angle pour que la distance entre les surfaces latérales (23) diminuent vers le fond (24) de la rainure.
